# EUROPÄISCHE PATENTSCHRIFT

(11) **EP 2 219 568 B1**
(45) Veröffentlichungstag und Bekanntmachung des Hinweises auf die Patenterteilung: **29.06.2011**
(21) Anmeldenummer: 08855267.4
(22) Anmeldetag: 14.11.2008
(51) Int. Cl.: A61F 5/01

(54) **SPRUNGGELENKORTHESENSYSTEM**
ORTHOTIC SYSTEM FOR AN ANKLE JOINT
SYSTÈME ORTHOPÉDIQUE POUR L'ARTICULATION DU TARSE

(30) Priorität: 28.11.2007 DE 102007057578
(43) Veröffentlichungstag der Anmeldung: 25.08.2010
(73) Patentinhaber: Otto Bock HealthCare GmbH, 37115 Duderstadt (DE)
(72) Erfinder: VOLLBRECHT, Matthias, 37412 Herzberg am Harz (DE); BRÜGGEMANN, Gert-Peter, 50858 Köln (DE); GÖSELE-KOPPENBURG, Andreas, 79540 Lörrach (DE); BEST, Raymond, 70180 Stuttgart (DE); ELLERMANN, Andree, 76275 Ettlingen (DE); SEMSCH, Hartmut, 70374 Stuttgart (DE); DRESSLER, Kai, 99086 Erfurt (DE); ALBASINI, Alfio, CH-6595 Riazzino (CH); LIEBAU, Christian, 38114 Braunschweig (DE)
(74) Vertreter: Stornebel, Kai
(86) Internationale Anmeldenummer: PCT/DE2008/001880
(87) Internationale Veröffentlichungsnummer: WO 2009/067982

(56) Entgegenhaltungen:
- DE-U1- 29 908 981
- US-A- 5 334 135
- US-A- 5 370 133

## Beschreibung

Die Erfindung betrifft ein Sprunggelenkorthesensystem zur Abstützung eines Sprunggelenkes mit einem formstabilen, flexiblen Grundkörper, der um die an das Sprunggelenk angrenzenden Gliedmaßen herum anlegbar ist und Verschlusseinrichtungen aufweist, über die der Grundkörper an den an das Sprunggelenk angrenzenden Gliedmaßen festlegbar ist. Ein solches System ist insbesondere zur Abstützung von Sprunggelenken geeignet, die eine Bänderschädigung aufweisen. Insbesondere Bänderrisse bzw. Bänderanrisse können mit einem solchen orthopädischen Stützsystem behandelt werden.

Stützeinrichtungen für Gelenke zur Behandlung von Verletzungen sind seit langer Zeit bekannt. Zur Behandlung von Bänderrissen im Sprunggelenk werden Winkelschienen über Klettverschlüsse an dem Unterschenkel und dem Fuß befestigt, um das Gelenk in einer vorgegebenen Haltung zu Fixieren.

Weiterhin sind Manschetten bekannt, die an den an das Gelenk angrenzenden Gliedmaßen festgelegt werden, um in einer bestimmten Bewegungsrichtung das Gelenk abzustützen oder eine solche Bewegung zu blockieren.

Aus der EP 0 876 130 B1 ist eine orthopädische Stützeinrichtung in Gestalt einer Handgelenks- oder Sprunggelenkorthese bekannt, bei der eine Halterung aus Kunststoff im Spritzgussverfahren ausgebildet ist und Bereiche unterschiedlicher Dicke aufweist. Zur Gewährleistung einer Anpassung an die Anatomie des Trägers der Stützeinrichtung sind flexible Bereiche ausgebildet, die eine geringere Dicke als diejenigen Bereiche aufweisen, die zur Abstützung des Gelenks vorgesehen sind. Verschlusseinrichtungen zum Festlegen der Stützeinrichtung an den Gliedmaßen sind einstückig an der Orthese angeformt und als Riemen mit Löchern ausgebildet, die durch D-Ringe hindurchgezogen und in hervorstehende Stifte eingehakt werden. Die Innenseite der Stützeinrichtung ist mit einer Polsterung versehen. Die Stützeinrichtung selbst weist harte Stabilisierungsbereiche auf.

Die US 5,334,135, das der nächste Stand der Technik darstellt, betrifft eine vorgeformte, federnde Kniebandage aus Schaummaterial, das in speziellen Bereichen komprimiert ist. Die komprimierten Bereiche sind in der Kniekehle angeordnet, so dass bei der Flexion Materialstauchungen verhindert werden.

Die US 5,370,133 beschreibt eine lmmobilisierungsorthese mit einer gleichförmigen, einstellbaren Kompression, wobei Dorsalflexion als auch Plantarflexion im angelegten Zustand verhindert wird. Dazu ist eine im Wesentlichen starre Laufsohle mit einem semiflexiblen Schuhbereich vorgesehen, der an der Oberseite der Laufsohle angeordnet ist. Ein Unterschenkelbereich ist starr an dem Schuhbereich angeordnet, sowohl in dem Schuhbereich als auch in dem Unterschenkelbereich sind Ventilationsöffnungen vorgesehen. Über Hebelschließeinrichtungen ist es möglich, die jeweils offenen Vorderseiten des Schuhbereiches und des Unterschenkelbereiches gleichmäßig zu spannen.

Aufgabe der vorliegenden Erfindung ist es, ein Sprunggelenkorthesensystem bereitzustellen, das eine flexible Behandlung bei Schädigungen des Stützapparates ermöglicht.

Erfindungsgemäß wird diese Aufgabe durch ein Sprunggelenkorthesensystem mit den Merkmalen des Anspruchs 1 gelöst. Vorteilhafte Ausgestaltungen und Weiterbildungen der Erfindung sind in den Unteransprüchen aufgeführt.

Das erfindungsgemäße Sprunggelenkorthesensystem mit einem formstabilen, flexiblen Grundkörper, der um die an das Gelenk angrenzenden Gliedmaßen herum anlegbar ist und Verschlusseinrichtungen aufweist, über die der Grundkörper an den an das Gelenk angrenzenden Gliedmaßen festlegbar ist, sieht vor, dass ein formstabiler, sich über das Gelenk erstreckender Außenrahmen an dem Grundkörper über einstellbare Befestigungsmittel abnehmbar festgelegt ist und der Außenrahmen sich über die an das Gelenk angrenzenden Gliedmaßen abstützt, Entlang von Falt- oder Biegelinien sind Materialschwächungen in dem Grundkörper eingebildet, um eine Anpassbarkeit an die individuellen Gegebenheiten zu ermöglichen. Der Außenrahmen ohne Gelenkeinrichtung dient dazu, das Gelenk in einer ersten Phase der Behandlung ruhig zu stellen, um den gewünschten Therapieerfolg zu erzielen. Nach einer Bänderschädigung im Sprunggelenk sollte das Gelenk über einen Zeitraum von beispielsweise einer Woche in einer vorgegebenen Stellung ruhiggestellt werden, um ein Zusammenwachsen der Bänder oder ein sich Zusammenziehen der Bänder zu ermöglichen. Nach der ersten Phase der Behandlung ist es wichtig, dass das Gelenk mobilisiert wird, um den Heilungsprozess zu beschleunigen und eine Versteifung des Gelenkes zu verhindern. Dazu wird das Stützsystem von dem Außenrahmen befreit, so dass während der Wachphase, üblicherweise tagsüber, das orthopädische Stützsystem ohne Außenrahmen getragen wird. Nur nachts wird der Außenrahmen wieder angelegt, um unwillkürliche Bewegungen und einen ungewollten Bewegungsumfang des Gelenkes während des Schlufens zu verhindern.

Um ein leichtes Anlegen und Abnehmen zu ermöglichen, ist der formstabile, das Gelenk festlegende Außenrahmen über einstellbare Befestigungsmittel an dem Grundkörper abnehmbar festgelegt. Die einstellbaren Befestigungsmittel ermöglichen eine Anpassung an sich abschwellende Gelenke nach einer Verletzung, so dass eine leichte Anpassbarkeit des Systems an den Heilungsverlauf möglich ist.

Die Erfindung sieht vor, dass der Grundkörper zumindest ein außenseitig angeordnetes Formschlusselement zur Festlegung zumindest eines Teils des Außenrahmens aufweist beispielsweise einer in Längsrichtung orientierten Schiene zur Anlage an einen Unterschenkel.

Das Formschlusselement kann als Tasche ausgebildet sein, so dass der Teil des Außenrahmens lediglich eingeschoben werden muss, um eine Festlegung an dem Grundkörper zumindest teilweise zu ermöglichen. Das Formschlusselement kann aufgenäht, aufgeklebt, aufgeklettet oder aufgeschweißt sein, wobei bei der Ausgestaltung als Tasche der Außenrahmen längsverschieblich in dem Formschlusselement bzw. in der Tasche gelagert ist, Entlang einer Längserstreckung eines Körpergliedes, beispielsweise eines Unterschenkels, kann ein Teil des Außenrahmens leicht und präzise eingeführt werden, so dass nur noch ein Fixierung entgegen der Längsverschieblichkeit zu erfolgen hat, um das Gelenk ruhig zu stellen. Das nicht an das Formschlusselement eingeführte oder daran festgelegte Teil des Außenrahmens kann auf andere Art und Weise an dem Grundkörper bzw. an der Gliedmaße befestigt sein.

Die Befestigungsmittel zum Festlegen des Außenrahmens an dem Grundkörper sind bevorzugt Klettverschlüsse, so dass eine nahezu stufenlose Einstellung und Anpassung an den Heilungsverlauf und die Dimensionen des abzustützenden Gelenkes ermöglicht sind.

Um eine weitere Option zur Behandlung zu haben, sind Aufnahmeeinrichtungen für Versteifungselemente an oder in dem Grundkörper ausgebildet. Diese Versteifungselemente, die als Schienen ausgebildet sind, verstärken die Formstabilität des Grundkörpers, so dass insbesondere während der zweiten Phase der Behandlung, nach dem Ablegen des Außenrahmens während des Tages eine Gelenkstabilisierung ermöglicht wird. Je nach Schädigung kann das Versteifungselement bzw. können die Versteifungselemente an vorbestimmten Aufnahmeeinrichtungen befestigt oder darin eingeführt sein, bei Sprunggelenksverletzungen insbesondere medial/lateral. Die Aufnahmeeinrichtungen für die Versteifungselemente sind bevorzugt als Taschen ausgebildet, so dass auch die Versteifungselemente leicht eingeschoben oder herausgenommen werden können.

Die Versteifungselemente können so angeordnet und ausgebildet sein, dass sie zumindest eine Bewegungsrichtung des Sprunggelenks einschränken oder blockieren, um bestimmte Bewegungen oder Bewegungsumfänge nicht oder nur eingeschränkt zuzulassen.

Sowohl der Außenrahmen als auch die Versteifungselemente können aus Kunststoff, Verbundmaterialien oder Leichtmetall ausgebildet sein, um den Träger des Stützsystems möglichst wenig zu beeinträchtigen.

Es kann vorgesehen sein, dass ein erster Teil des Außenrahmens sich entlang des Unterschenkels und ein zweiter Teil des Außenrahmens sich im Plantarbereich des Fußes erstreckt, um den Fuß bzw. ein Teil des Fußes aufnehmen zu können. Der erste und der zweite Teil des Außenrahmens sind dabei bevorzugt in einem annähernd rechten Winkel angeordnet, um den Fuß und den Unterschenkel in dieser Stellung zueinander zu stabilisieren und eine Heilung zu fördern. Bei einem Außenbandschaden ist der zweite Teil, also die Fußschale, in einer Pronationsstellung zu dem Unterschenkelteil ausgerichtet. Der zweite Teil, der als Fußschale ausgebildet sein kann, ist bevorzugt in Dorsalextension und Pronation biegbar, während die als Zuggurte ausgebildeten Befestigungseinrichtungen zur Festlegung des Außenrahmens an dem Grundkörper oder separate Zuggurte die Supination und die Dorsalflexion einschränken oder blockieren. Der zweite Teil des Außenrahmens, also die Fußschale, die sich bis unter den Vorderfuß erstrecken kann, ist so ausgebildet, dass sie auch bei weiter Umfassung der Fußsohle bis unter den Vorderfuß das erste Metatarsalköpfchen freilässt. Ein weiterer Zuggurt kann als ein um den Vorderfuß schlingbares Fixierungsmittel ausgebildet sein.

Bei einer Sprunggelenksabstützung kann der Plantarbereich des Grundkörpers durch zwei einander gegenüberliegende Grundkörperabschnitte ausgebildet sein, die über zumindest ein Verbindungselement, das Klettelemente aufweist, miteinander gekoppelt sind. Dadurch ist es möglich, den Grundkörper aus einem flächigen Zuschnitt auszubilden und an sich verändernde Ausmaße des Fußes bzw. des Sprunggelenkes anzupassen. Das Verbindungselement kann als separater Zuschnitt, insbesondere als beidseitiger Klettzuschnitt ausgebildet sein, um bei einer Ausgestaltung des Grundkörpers mit einer Flauschschicht auf der Außenseite eine möglichst flexible Festlegung ermöglichen zu können.

Im Bereich der Achillessehne des angelegten Grundkörpers sind Einschnitte vorgesehen, durch die Anpassungsflügel ausgebildet werden, um den Tragekomfort zu verbessern.

Die Verschlusseinrichtungen sind ebenfalls als Klettelemente, insbesondere als Mikroklettelemente ausgebildet, um eine nahezu stufenlose Anpassung zu ermöglichen. Die Verschlusseinrichtungen sind dabei an dem Grundkörper befestigt, insbesondere als Gurte ausgebildet, und in Durchbrechungen des Grundkörpers unter Ausbildung einer Schlaufe vernäht.

Zur Erhöhung des Tragekomforts ist der Grundköper mit einer Polsterung in Gestalt einer Flauschschicht versehen, ebenfalls kann der Grundkörper außenseitig vollständig mit einer Flauschschicht beschichtet sein, um die Verschlusseinrichtungen nahezu unbegrenzt an dem Grundkörper festlegen zu können. Ebenfalls können daran die Befestigungsmittel zur Befestigung des Außenrahmens angeordnet werden, wobei bei einer vollständigen außenseitigen Flauschbeschichtung keinerlei vorgegebene Befestigungspunkte eingehalten werden müssen. Die Art und Weise der Befestigung und zusätzliche Stabilisierung der Gliedmaße an dem Außenrahmen kann individuell erfolgen, so dass auf die individuellen Gegebenheiten und Befindlichkeiten Rücksicht genommen werden kann.

Die Polsterung und die Flauschschicht sind miteinander verklebt oder verschweißt, insbesondere laserverschweißt oder hochfrequenzverschweißt. Der Grundkörper kann Ausnehmungen innerhalb seiner Kontur aufweisen, die insbesondere im Urformverfahren und durch ein Trennverfahren in den Grundkörper eingebracht sind. Sofern die Flauschschicht und die Polsterung an dem Grundkörper vorgesehen sind, können diese durch die Ausnehmungen hindurch miteinander verschweißt oder verklebt sein und dadurch an dem Grundkörper fixiert werden. Der Grundkörper kann mehrlagig aufgebaut und insbesondere spiegelsymmetrisch ausgebildet sein.

Ein Fixiergurt kann mittels Klettelementen an der Flauschschicht befestigt sein, um den Grundkörper an den Gliedmaßen festlegen zu können.

Die Befestigungsmittel können als unelastische oder halbelastische Zuggurte ausgebildet sind, die an dem Grundkörper festlegbar sind.

Nachfolgend werden Ausführungsbeispiele der Erfindung anhand der beigefügten Figuren näher erläutert. Es zeigen:
- Figur 1 -: eine Draufsicht auf einen Grundkörperzuschnitt;
- Figur 2 -: eine Untenansicht eines angelegten Grundkörpers;
- Figur 3 -: eine Rückenansicht eines angelegten Grundkörpers;
- Figur 4 -: eine perspektivische Schrägdraufsicht eines angelegten Grundkörpers mit Fixiergurt;
- Figuren 5 und 6 -: perspektivische Schrägdraufsichten einen angelegten Grundkörpers ohne Außenrahmen;
- Figuren 7 und: 8 - Seitenansichten eines angelegten Grundkörpers ohne Außenrahmen;
- Figuren 9 und 10 -: perspektivische Schrägdraufsichten einen angelegten Grundkörpers mit Außenrahmen;
- Figuren 11 und 12 -: Seitenansichten eines angelegten Grundkörpers mit Außenrah- men; sowie
- Figur 13: eine Untenansicht mit Außenrahmen.

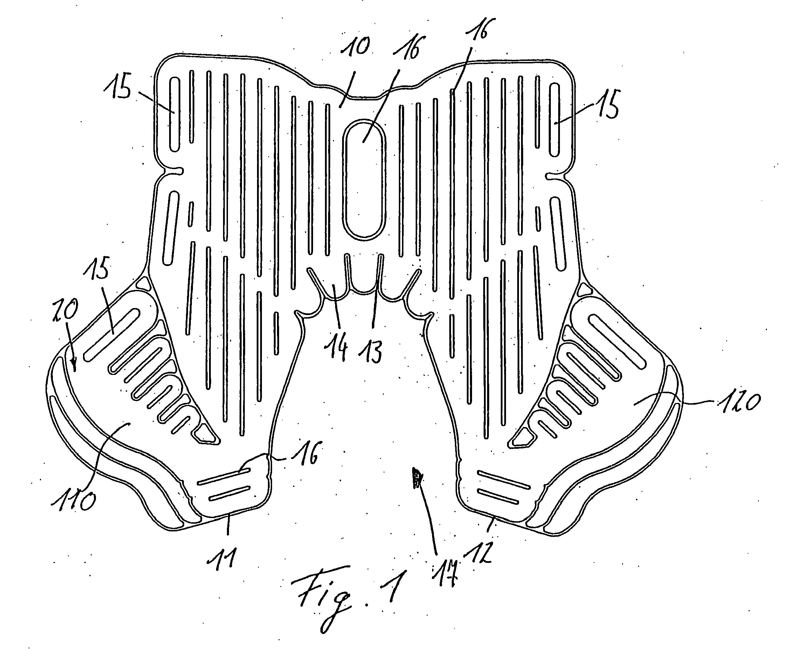

Figur 1 zeigt in Draufsicht einen Zuschnitt einer Stützeinrichtung 1 mit einem Grundkörper 10, der außenseitig mit einer Flauschschicht 20 ganzflächig beschichtet ist. Der Grundkörper 10 in dem dargestellten Ausführungsbeispiel ist zur Verwendung in einer Stützeinrichtung in Gestalt einer Sprunggelenksorthese vorgesehen und ist spiegelsymmetrisch aufgebaut. Zur Befestigung des Grundkörpers 10 an dem Bein eines Nutzers wird der Grundkörper 10 mit seiner Spiegellinie im Bereich der Achillessehne angelegt und nach vorne in Richtung Schienenbein umgeschlagen. Zwei Seitenbereiche 110, 120 werden um den Fuß herum gelegt und einander gegenüberliegende Grundkörperabschnitte 11, 12, liegen im entsprechend umgeklappten Zustand im Plantarbereich des Orthesennutzers einander gegenüber. Zur Gewährleistung einer erhöhten Flexibilität in den Faltbereichen und zur verbesserten Anschmiegung der Stützeinrichtung an die Anatomie des Nutzers sind Ausnehmungen 16 innerhalb des Grundkörpers 10 vorgesehen, die eine Materialschwächung bewirken und dadurch ein leichteres Biegen gestatten. Die Ausnehmungen 16 innerhalb des aus einem flächigen Kunststoff hergestellten Grundkörpers 10 sind von der Flauschschicht 20 und der nicht dargestellten, innen liegenden Polsterschicht bedeckt, so dass sich eine im Wesentlichen geschlossene Oberfläche ausbildet. Dadurch können Kleidungsstücke oder dergleichen sich nicht verhaken. Im Bereich der Ausnehmungen 16 sind sowohl die innen liegende Polsterschicht als auch die außen liegende Flauschschicht miteinander verbunden, vorzugsweise verklebt oder verschweißt, insbesondere hochfrequenzverschweißt.

Um den Grundkörper 10 herum sind die Flauschschicht 10 und die Polsterschicht ebenfalls verschweißt, so dass der Grundkörper 10 vollständig von einer Stoff- bzw. Gewebeschicht umgeben ist. Die außen liegende Flauschschicht 20 ermöglicht es Klettelementen ein Widerlager zu finden, so dass an beliebiger Stelle der Außenseite des Grundkörpers 10 eine Fixierung von Gurten oder dergleichen möglich ist.

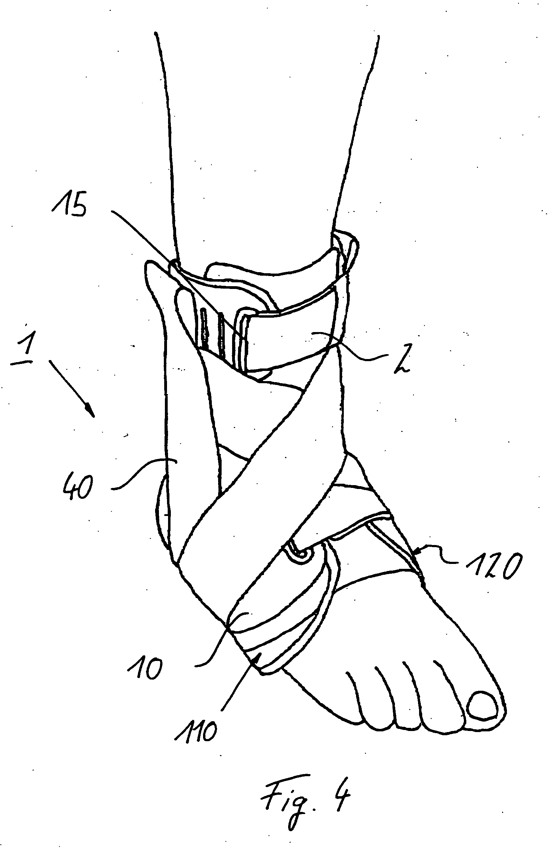

Zur Festlegung der Stützeinrichtung an dem Unterschenkel bzw. dem Fuß werden Verschlusseinrichtungen, die in der Figur 4 dargestellt sind, durch Durchbrechungen 15, die nicht mit einer Flauschschicht 20 oder einer Polsterschicht versehen sind, hindurchgeführt und an einer Seite vernäht. Dann werden die mit einem Klettelement versehenden Verschlusseinrichtungen 2 durch die gegenüberliegende Ausnehmung 15 bzw. Durchbrechung hindurch geführt und zurückgeschlagen, um anschließend auf einem Bereich der Flauschschicht 20 verhakt zu werden. Zwei übereinander angeordnete Durchbrechungen 15 dienen zur Festlegung an dem Unterschenkel, eine dritte Durchbrechung 15 im Dorsalbereich des Fußes dient zur fußseitigen Fixierung.

Eine langlochartige Ausnehmung 16 ist im Bereich der Achillessehne innerhalb des Grundkörpers 10 ausgebildet und erleichtert eine Bewegung des Fußes, insbesondere eine Plantarflexion, wohingegen die Beweglichkeit des Sprunggelenkes um eine Schwenkachse in Sagittalrichtung durch den Grundkörper 10 und gegebenenfalls weitere Stützeinrichtungen verhindert wird.

Weiterhin sind im Bereich der Achillessehne oberhalb eines Ausschnittes 17 für die Ferse Einschnitte 13 ausgebildet, die Anpassungsflügel 14 erzeugen, mit denen eine autoadaptive Anpassung und eine erhöhte Flexibilität innerhalb des Achillessehnenbereiches gewährleistet wird.

Um eine gezielte Einstellung der Flexibilität der Stützeinrichtung gewährleisten zu können, sind an dem Grundkörper 10, der bevorzugt einstückig und plattenartig ausgebildet ist, unterschiedliche Materialstärken vorgesehen, wobei Materialanhäufungen in Bereichen erhöhter Belastung vorgesehen sind. Materialschwächungen hingegen sind in denjenigen Bereichen vorgesehen, in denen gewollte Biegungen zur Anpassung an die Anatomie eines Nutzers erfolgen.

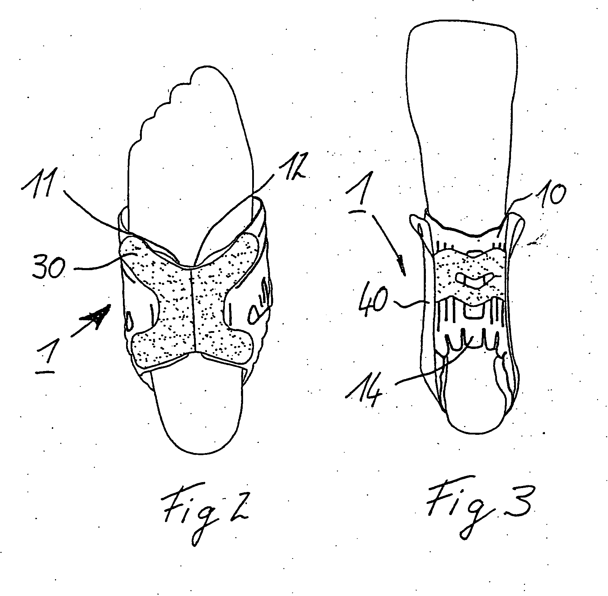

In der Figur 2 ist in Unteransicht eine angelegte Stützeinrichtung in Gestalt einer Sprunggelenksorthese gezeigt. Im Plantarbereich sind die beiden Grundkörperabschnitte 11, 12 einander gegenüberliegend ausgerichtet und über ein Verbindungselement 30, das Klettelemente aufweist, miteinander gekoppelt. Das Verbindungselement 30 ist als separater, einteiliger Zuschnitt ausgebildet. Das Verbindungselement 30 besteht dabei vorzugsweise aus einem relativ steifen Gewebe, das zumindest einseitig mit Kletteinrichtungen zum Verhaken mit der außenliegenden Flauschschicht 20 versehen ist. Durch die relative Steifigkeit des Verbindungselementes 30 wird eine Plantarstabilität der Stützeinrichtung 1 erzeugt. Außenseitig kann das Verbindungselement 30 entweder mit einer Flauschschicht oder ebenfalls mit einer Klettschicht, insbesondere Mikroklettschicht, versehen sein.

Durch die separate Ausbildung des Verbindungselementes 30 kann der in wenigen Standardgrößen zu fertigende Grundkörper 10 einfach an unterschiedliche anatomische Gegebenheiten angepasst werden. Nach erfolgter einmaliger Anpassung kann das Verbindungselement 30 an Ort und Stelle verbleiben, ein Anlegen bzw. Ablegen der Stützeinrichtung 1 erfolgt durch Öffnen und Schließen der Verschlusseinrichtungen 2. Darüber hinaus ist es möglich, dass die Stützeinrichtung 1 an sich verändernde Anatomien angepasst wird, beispielsweise bei abschwellenden Gliedmaßen durch ein Engerstellen eines gegebenenfalls vorhandenen Spaltes zwischen den Grundkörperabschnitten 11, 12.

In der Figur 3 ist in Rückansicht eine angelegte Stützeinrichtung 1 mit dem Grundkörper 10 dargestellt. Die Anpassungsflügel 14 sind deutlich zu erkennen. Zur Erhöhung der Stabilität ist an der Außenseite des Grundkörpers 10 auf der Flauschschicht 20 ein Fixiergurt 40 über Klettverbindungen befestigt. Der Fixiergurt 40 ist biegsam, jedoch nicht elastisch, so dass nach einmaligem Anlegen eine signifikante Steifigkeitserhöhung bewirkt wird. Die Position des Fixiergurtes 40 oder mehrerer Fixiergurte kann frei gewählt werden, da die gesamte Oberfläche des Grundkörpers 10 mit der Flauschschicht 20 bedeckt ist. Das dargestellte Ausführungsbeispiel der orthopädischen Stützeinrichtung 1 besteht somit aus dem Grundkörper 10 mit der nicht zu erkennenden Polsterung und der außenseitig aufgebrachten Flauschschicht, einem plantar angeordneten Verbindungselement 30 und dem Fixiergurt 40, der mit Klettelementen versehen ist. Sofern keine zusätzliche Fixierung notwendig ist, kann die Stützeinrichtung auch ohne Fixiergurt 40 ausgebildet sein und aus dem Grundkörper 10 mit den außenseitig und innenseitig angebrachten Textilschichten und dem Verbindungselement 30 bestehen.

In der Figur 4, die eine angelegte Stützenrichtung 1 in perspektivischer Schrägdraufsicht zeigt, sind neben dem Grundkörper 10 mit den Fußbereichen 110, 120 und der Durchrechung 15 für eine Verschlusseinrichtung 2 die Anordnung des Fixiergurtes 40 gezeigt, der sowohl dorsal als auch plantar geführt ist und zur Fixierung des Sprunggelenkes, beispielsweise nach einer Bänderruptur, eingesetzt werden kann. Mit zunehmender Beweglichkeit des Sprunggelenkes kann eine lockerere Wicklung vorgenommen oder der Fixiergurt 40 weggelassen werden.

Statt einer Durchbrechung 15 können auch D-Ringe an dem Grundkörper 10 befestigt werden, um die Verschlusseinrichtungen 2 zu befestigen bzw. durchzuführen.

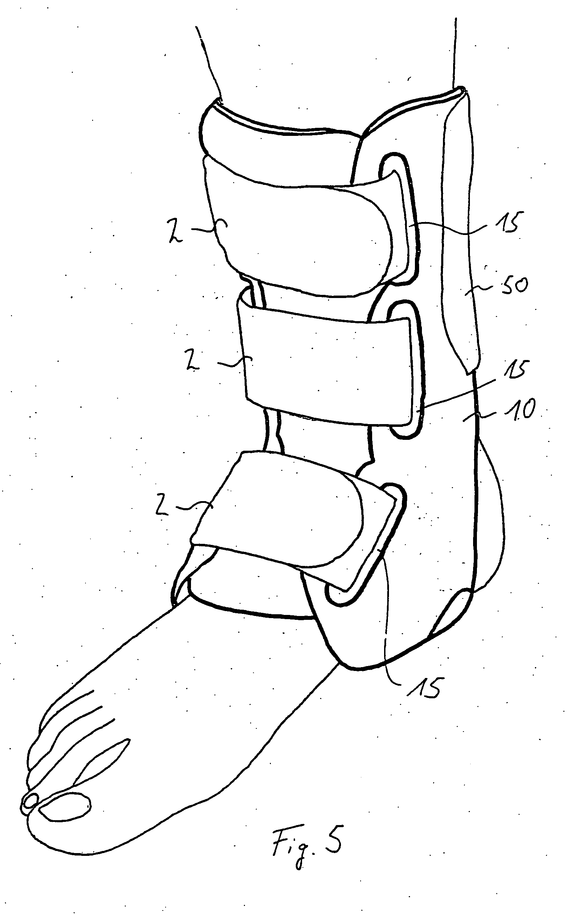

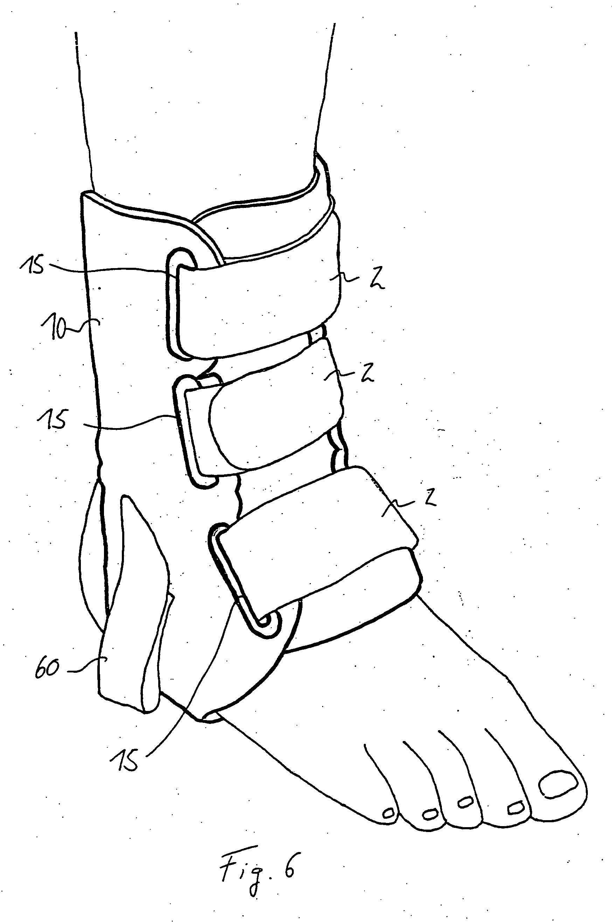

In den Figuren 5 und 6 ist jeweils in einer perspektivischen Schrägdraufsicht ein Grundkörper 10 in einem angelegten Zustand als Abstützung eines Sprunggelenkes dargestellt. Über als Klettriemen ausgebildete Verschlusseinrichtungen 2 wird der Grundkörper 10 sowohl an dem Unterschenkel als auch an dem Fuß festgelegt und stabilisiert das Sprunggelenk. An der Außenseite des Grundkörpers 10 ist oberhalb des Sprunggelenkes auf der Medialseite ein Formschlusselement 50 in Gestalt einer Aufnahmetasche angeordnet, in die ein Außenrahmen 70 einführbar ist. Das Formschlusselement 50 ist unten offen, so dass der Außenrahmen 70 von unten eingeschoben werden kann und eine Beugebewegung des Unterschenkels relativ zu dem Fuß verhindert, wenn der Fuß daran fixiert ist. Die Verschlusseinrichtungen 2 sind durch Ausnehmungen 15 innerhalb des Grundkörpers 10 geführt und können in ihrer Weite einstellbar ausgebildet sein, so dass sich eine Anpassung an die individuellen Gegebenheiten des Trägers des Stützsystems bzw. des Grundkörpers 10 erreichen lässt. Der Grundkörper 10 kann allseitig abgepolstert und mit einer Flauschschicht versehen sein, um den Tragekomfort zu erhöhen und Widerhalt für Hakenelemente eines Klettverschlusses bieten zu können. Sowohl an der Dorsalseite des Fußes als auch an der Frontalseite des Schienbeines sind Polsterungen angeordnet, die an dem Grundkörper 10 festgelegt sein können, so dass die Verschlusseinrichtungen 2 nicht unmittelbar auf der Haut des Stützsystemträgers aufliegen. Neben der Ausbildung des Formschlusselementes 50 als Einschubtasche kann dieses auch eine andere gestalt aufweisen, beispielsweise als Vorsprung, Druckknopf, Lasche, Riemen oder als Klettverschluss ausgebildet sein.

In der Figur 6 ist an der Lateralseite ein Befestigungsmittel 60 in Gestalt eines Klettbandes dargestellt, das an einem Ende an dem Grundkörper 10 festgelegt ist, beispielsweise angenäht, angeklebt oder angeschweißt, während das andere, freie Ende an einer außenseitigen Flauschschicht des Grundkörpers 10 befestigt ist. Zur Festlegung eines Außenrahmens kann das abnehmbare Ende des Befestigungsmittels 60 durch eine Ausnehmung hindurchgezogen und dann wieder an dem Grundkörper 10 festgelegt werden, um so in dem Plantarbereich eine Fixierung eines Außenrahmens 70 zu ermöglichen. Statt einer Befestigung über eine Naht kann das Klettband auch über eine Klettverbindung an dem Grundkörper 10 festgelegt werden.

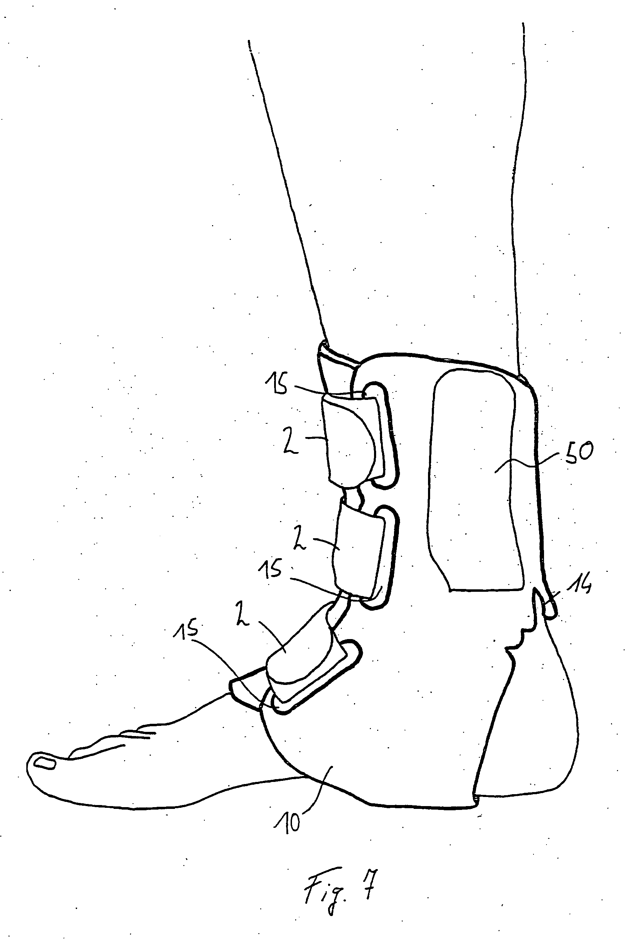

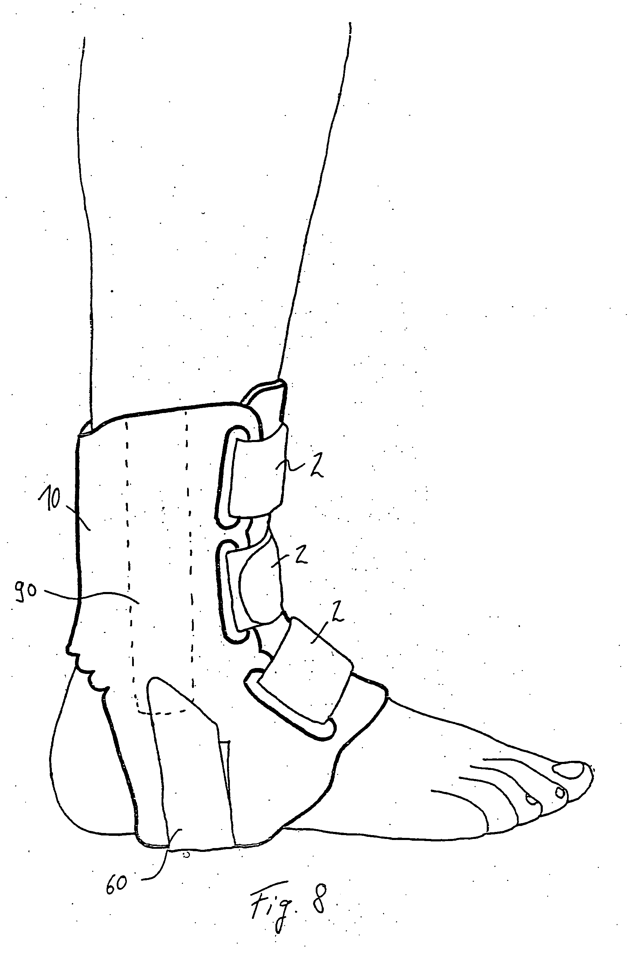

In den Figuren 7 und 8 sind Seitenansichten des angelegten Grundkörpers 10 gezeigt, aus denen einerseits die Anordnung des Formschlusselementes 50 in Gestalt einer Einschubtasche auf der Medialseite und andererseits die Anordnung einer Aufnahmeeinrichtung 90 auf der Lateralseite für nicht dargestellte Versteifungselemente, beispielsweise Schienen oder dergleichen, zu entnehmen sind. Grundsätzlich kann es vorgesehen sein, dass der Grundkörper 10 symmetrisch ausgebildet ist, so dass er sowohl für den linken als auch für den rechten Fuß anwendbar ist. Dazu sind sowohl Formschlusselemente 50 als auch Aufnahmeeinrichtungen 90 für Versteifungselemente beiderseitig angeordnet, also lateral und medial. Dadurch ist es möglich, den Fuß sowohl in Pronations- als auch in Supinationsstellung, rechts wie links zu fixieren, indem ein entsprechender Außenrahmen 70 an dem Grundkörper 10 festgelegt wird. Dazu kann auch das Befestigungsmittel 60 sowohl medial als auch lateral vorgesehen sein. Sofern das Befestigungsmittel 60 nur über Klettverschlüsse an dem Grundkörper 10 festgelegt ist, bedarf es keiner doppelten Ausführung. Eine Fixierung ider der gewünschten Pronationsstellung mit ggf. Dorsalextension kann über die Befestigungsmittel 60, 80 oder separate Zuggurte, die unelastisch oder halbelastisch ausgebildet sein können, erfolgen.

Die Aufnahmeeinrichtung 90 für Versteifungselemente kann über das Gelenk hinaus reichen, um eine zusätzliche Versteifungswirkung bereitstellen zu können. Bevorzugt ist die Aufnahmeeinrichtung 90 bzw. sind die Aufnahmeeinrichtungen 90 als Taschen ausgebildet, die beispielsweise zwischen einer äußeren Flauschschicht und einem Kunststoffgrundmaterial eingearbeitet sind. Ebenfalls ist es möglich, dass die Aufnahmeeinrichtungen 90 für Versteifungselemente von dem Formschlusselement 50 abgedeckt werden, also dass mehrere Taschen übereinander angeordnet sind, in die einerseits Versteifungselemente und andererseits der Außenrahmen eingeführt werden kann. Die Aufnahmeeinrichtungen 90 für die Versteifungselemente sind verschließbar, so dass die Versteifungselemente, insbesondere Versteifungsschienen, nicht aus den Aufnahmeeinrichtungen 90 unbeabsichtigt herausgeschoben werden können.

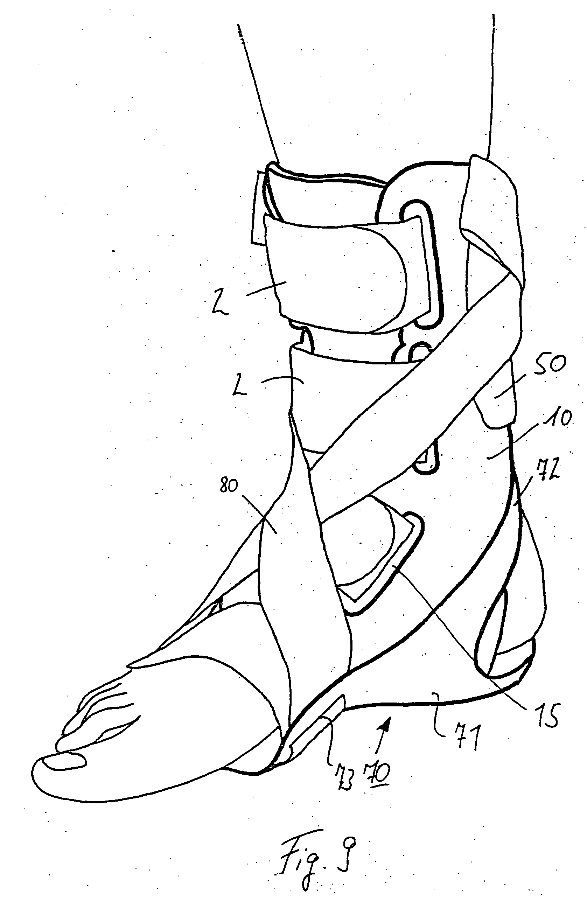

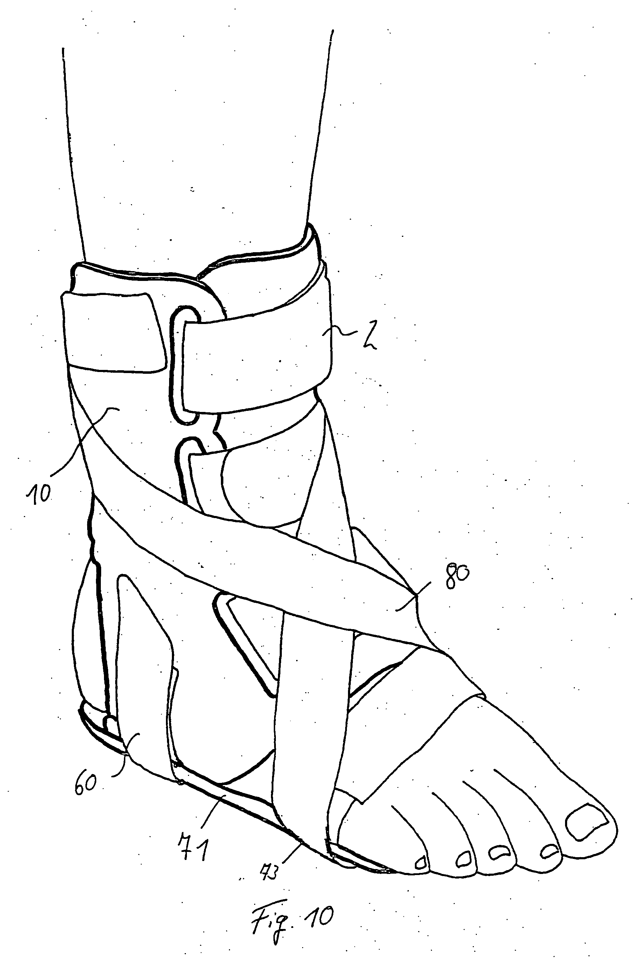

In den Figuren 9 und 10 sind perspektivische Schrägdarstellungen eines angelegten Stützsystems bestehend aus einem Grundkörper 10 und einem daran festgelegten Außenrahmen 70 gezeigt, die aneinander über Befestigungsmittel 80, 60 in Gestalt von Klettverschlussbändern fixiert sind. Der Außenrahmen 70 ist dabei einstückig ausgebildet und weist einen Plantarbereich 71 sowie einen Unterschenkelbereich 72 auf. Der Unterschenkelbereich 72 ist in das Formschlusselement 50 in Gestalt einer Einschubtasche eingeführt. Das Befestigungsmittel 80 in Gestalt eines flexiblen, unelastischen Klettbandes ist durch Schlitze 73 im Plantarbereich 71 des Außenrahmens 70 hindurchgeführt und dann an der Oberseite des Fußes kreuzweise entlanggeführt, um dann an der Außenseite des Grundkörpers 10 im Unterschenkelbereich festgelegt zu werden. Durch die kreuzweise Führung des Befestigungsmittels 80 über dem Fußrücken, der medialen und lateralen Anordnung an dem Außenrahmen 70 und der vollflächigen Anlage aufgrund der Flauschschicht auf der Außenseite des Grundkörpers 10 und auf der Außenseite des Formschlusselementes 50 kann eine weitgehende Stabilisierung des Sprunggelenkes und Immobilisierung erfolgen. Die Befestigungsmittel 80 können zudem im Wadenbereich des Grundkörpers 10 festgelegt werden. In der Figur 10 ist angedeutet, dass das zweite Befestigungsmittel 60 ebenfalls durch nicht dargestellte Schlitze entlanggeführt ist und im Knöchelbereich den zweiten Teil 71 des Außenrahmens 70 an dem Grundkörper 10 festlegt. Dadurch kann beispielsweise der Fuß in der gewünschten Stellung, Pronationsstellung oder Supinationsstellung, gehalten werden.

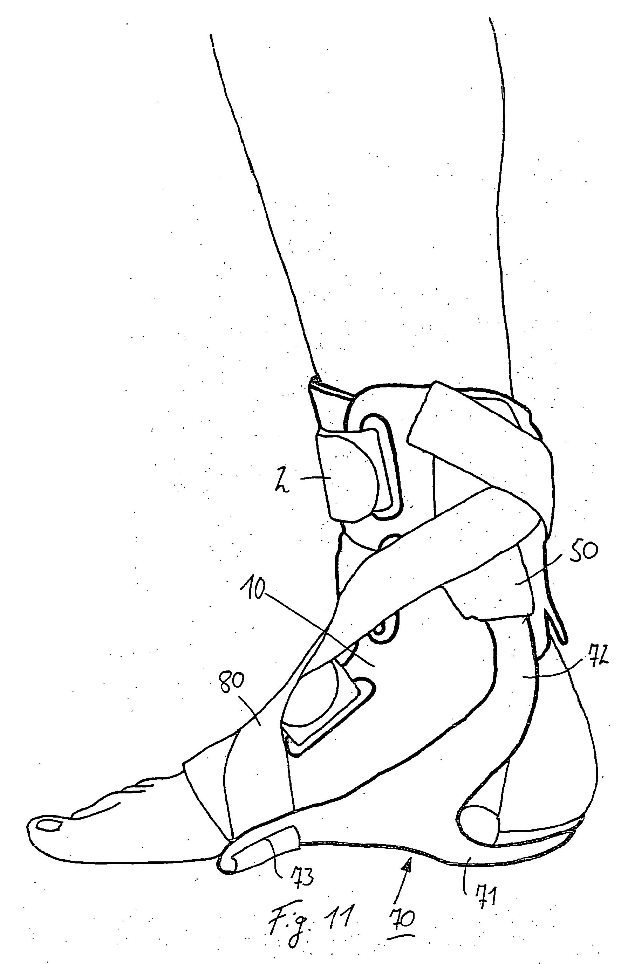

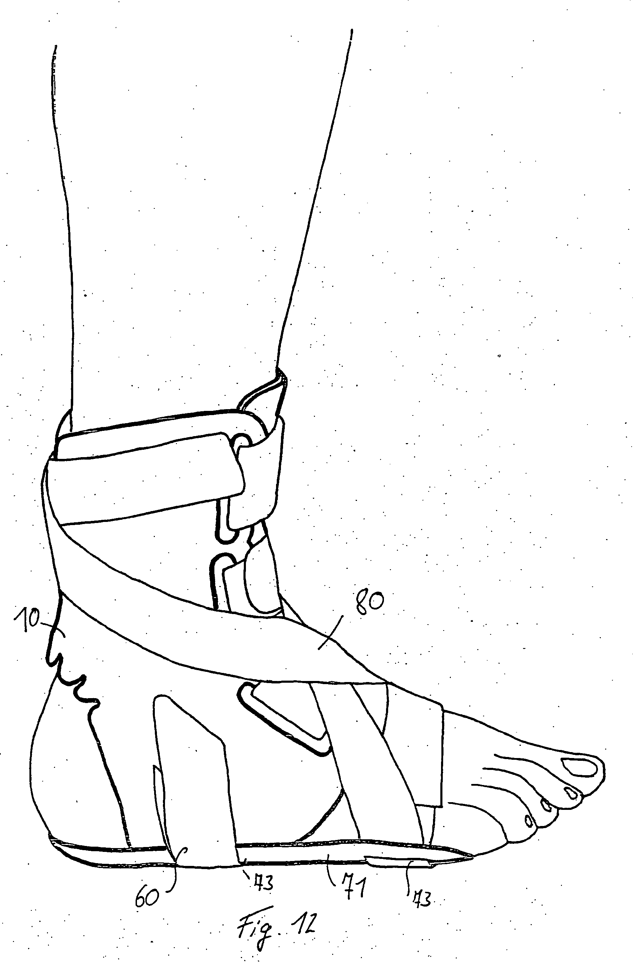

Die Figuren 11 und 12 zeigen die Anordnung der Befestigungsmittel 60, 80 und die Festlegung des Außenrahmens 70, insbesondere des dem Unterschenkel zugeordneten ersten Teils 72 in dem Formschlusselement 50. Der Außenrahmen 70 ist einstückig ausgebildet und weist eine Krümmung auf, so dass der erste Teil 72 in Gehrichtung hinter dem Knöchel verläuft, um eine möglichst angenehme Tragweise bereitstellen zu können. Durch das Einschieben des ersten Teils 72 in die Tasche 50 und Festlegen über die Befestigungsmittel 60, 80 kann das Stützsystem aus Grundkörper 10, Außenrahmen 70 und gegebenenfalls Versteifungselementen leicht an die Bedürfnisse der Therapie angepasst werden.

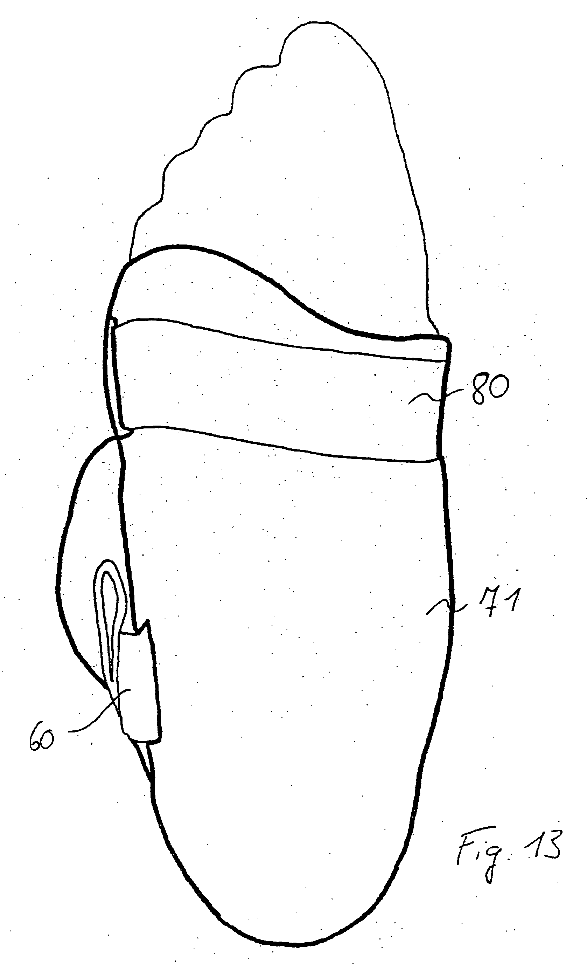

In der Figur 13 ist eine Untenansicht des angelegten Stützsystems gezeigt, in der der Plantarteil 71 des Außenrahmens 70 zu erkennen ist. Das Befestigungsmittel 80 ist unterhalb der starren Fußschale entlang geführt, so dass durch die auf der Fußoberseite gekreuzte Führung des Befestigungsmittels 80 der Fuß an der Fußschale gehalten wird. Über das zweite Befestigungsmittel 60 kann im vorliegenden Fall eine Pronationsstellung des Fußes durch eine entsprechende Fixierung der Fußschale 71 an dem Grundkörper 10 realisiert werden. Das orthopädische Stützsystem, wie abgebildet, besteht also aus einem relativ flexiblen Grundkörper 10, bevorzugt mit integrierten Versteifungselemente, einem Außenrahmen 70 sowie Befestigungsmitteln 60, 80, die in Gestalt einer Bandage eine Kombination aus Grundkörper 10 und Außenrahmen 70 ermöglichen. Diese Bandierung über die Befestigungsmittel 60, 80 führt zu einer weiteren Stabilisierung des Gelenkes.

Bei dem System handelt sich somit um eine aufrüstbare oder abrüstbare Orthese mit zumindest einem formstabilen, elastischen Einsatz in Gestalt eines Grundkörpers 10 und einem Außenrahmen 70 sowie gegebenenfalls weiteren Versteifungselementen.

## Patentansprüche

1. Sprunggelenkorthesensystem mit einem formstabilen, flexiblen Grundkörper (10), der um die an das Gelenk angrenzenden Gliedmaße herum anlegbar ist und Verschlusseinrichtungen (2) aufweist, über die der Grundkörper (10) an den an das Gelenk angrenzenden Gliedmaßen festlegbar ist, wobei ein formstabiler, sich über das Sprunggelenk erstreckender Außenrahmen (70) an dem Grundkörper (10) über einstellbare Befestigungsmittel (60, 80) abnehmbar festgelegt ist und der Außenrahmen (70) sich über die an das Sprunggelenk angrenzenden Gliedmaßen abstützt, **dadurch gekennzeichnet, dass** entlang von Falt- oder Biegelinien (17) Materialschwächungen in dem Grundkörper (10) ausgebildet sind und der Grundkörper (10) zumindest ein außenseitig angeordnetes Formschlusselement (50) zur Festlegung zumindest eines Teiles (72) des Außenrahmens (70) aufweist.

2. Sprunggelenkorthesensystem nach Anspruch 1, **dadurch gekennzeichnet, dass** das Formschlusselement (50) als Tasche ausgebildet und aufgenäht, aufgeklebt, aufgeklettet oder aufgeschweißt ist.

3. Sprunggelenkorthesensystem nach Anspruch 1 oder 2, **dadurch gekennzeichnet, dass** der Außenrahmen (70) längsverschieblich in dem Formschlusselement (50) gelagert ist.

4. Sprunggelenkorthesensystem nach einem der voranstehenden Ansprüche, **dadurch gekennzeichnet, dass** Aufnahmeeinrichtungen (90) für Versteifungselemente an oder in dem Grundkörper (10) als Taschen ausgebildet sind.

5. Sprunggelenkorthesensystem nach Anspruch 4, **dadurch gekennzeichnet, dass** die Versteifungselemente zumindest eine Bewegungsrichtung des Sprunggelenks einschränkend angeordnet sind.

6. Sprunggelenkorthesensystem nach einem der voranstehenden Ansprüche, **dadurch gekennzeichnet, dass** ein erster Teil (72) des Außenrahmens (70) sich entlang eines Unterschenkels und ein zweiter Teil (71) des Außenrahmens (70) sich im Plantarbereich eines Fußes erstreckt, dass der erste und der zweite Teil (72, 71) in einem annähernd rechten Winkel zueinander angeordnet sind und dass der zweite Teil (71) das erste Metatarsalköpfchen freilässt.

7. Sprunggelenkorthesensystem nach Anspruch 6, **dadurch gekennzeichnet, dass** der zweite Teil (71) in eine Pronationsstellung und/oder Dorsalextension zum ersten Teil (72) biegbar ist.

8. Sprunggelenkorthesensystem nach einem der voranstehenden Ansprüche, **dadurch gekennzeichnet, dass** im Bereich der Achillessehne Einschnitte (13) im Grundkörper (10) eingebracht sind, durch die Anpassungsflügel (14) ausgebildet werden.

9. Sprunggelenkorthesensystem nach einem der voranstehenden Ansprüche, **dadurch gekennzeichnet, dass** der Grundkörper (10) eine Polsterung in Gestalt einer Flauschschicht aufweist, dass der Grundkörper (10) außenseitig vollständig mit einer Flauschschicht (20) beschichtet ist und die Polsterung und/oder die außenseitige Flauschschicht (20) auf den Grundkörper (10) aufgeklebt oder aufgeschweißt sind.

10. Sprunggelenkorthesensystem nach Anspruch 9, **dadurch gekennzeichnet, dass** der Grundkörper (10) Ausnehmungen (16) innerhalb seiner Kontur aufweist, durch die die Polsterung und die Flauschschicht (20) miteinander verklebt oder verschweißt, insbesondere laserverschweißt oder hochfrequenzverschweißt sind.

11. Sprunggelenkorthesensystem nach einem der voranstehenden Ansprüche, **dadurch gekennzeichnet, dass** der Grundkörper (10) spiegelsymmetrisch ausgebildet ist.

12. Sprunggelenkorthesensystem nach einem der voranstehenden Ansprüche, **dadurch gekennzeichnet, dass** die Befestigungsmittel (60, 80) als unelastische oder halbelastische Zuggurte ausgebildet sind, die an dem Grundkörper (10) festlegbar sind.

## Claims

1. Orthotic system for an ankle joint with an inherently stable, flexible base body (10), which can be placed around the limbs adjoining the joint and which has locking devices (2) by means of which the base body (10) can be fixed against the limbs adjoining the joint, wherein an inherently stable external frame (70) extending over the ankle joint is fixed to the base body (10) in a detachable fashion by means of adjustable attachment means (60, 80) and the external frame (70) is braced by the limbs adjoining the ankle joint, **characterized in that** the material weakenings are formed in the base body (10) along folding or bending lines and the base body (10) has at least one interlocking element (50) arranged on the outside for fixing at least one part (72) of the external frame (70).

2. Orthotic system for an ankle joint as claimed in claim 1, **characterized in that** the interlocking element (50) is sewn on, stuck on, attached by a hook-and-loop fastener or welded on and designed as a pocket.

3. Orthotic system for an ankle joint as claimed in claim 1 or 2, **characterized in that** the external frame (70) is mounted in the interlocking element (50) such that it can be displaced in the longitudinal direction.

4. Orthotic system for an ankle joint as claimed in one of the preceding claims, **characterized in that** receptacle devices (90) for stiffening elements are formed as pockets on or in the base body (10).

5. Orthotic system for an ankle joint as claimed in claim 4, **characterized in that** the stiffening elements are arranged to limit at least one movement direction of the ankle joint.

6. Orthotic system for an ankle joint as claimed in one of the preceding claims, **characterized in that** a first part (72) of the external frame (70) extends along the a lower leg and a second part (71) of the external frame (70) extends on the plantar region of foot, that the first and the second part (72, 71) are arranged at a substantially right angle with respect to one another and that the second part (71) does not cover the first metatarsal head.

7. Orthotic system for an ankle joint as claimed in claim 6, **characterized in that** the second part (71) can be bent into a pronation and/or dorsal extension with respect to the first part (72).

8. Orthotic system for an ankle joint as claimed in one of the preceding claims, **characterized in that** incisions (13) are introduced in the base body (10) in the region of the Achilles tendon, with adjustments wings (14) being formed by the incisions.

9. Orthotic system for an ankle joint as claimed in one of the preceding claims, **characterized in that** base body (10) has a cushioning in the form of a layer of fleece, that the base body (10) is completely coated by a layer of fleece (20) on the outside and that the cushioning and/or the layer of fleece (20) on the outside are bonded or welded onto the base body (10).

10. Orthotic system for an ankle joint as claimed in claim 9, **characterized in that** the base body (10) has cuts (16) within its contour through which the cushioning and the layer of fleece (20) are bonded or welded to one another, in particular, they are laser welded or high-frequency welded to one another.

11. Orthotic system for an ankle joint as claimed in one of the preceding claims, **characterized in that** the base body (10) has a mirror-symmetric design.

12. Orthotic system for an ankle joint as claimed in one of the preceding claims, **characterized in that** the attachment means (60, 80) are designed as inelastic or semi-elastic tension belts, which can be fixed to the base body (10).

## Revendications

1. Système d'orthèse pour l'articulation du tarse comprenant un corps de base (10) flexible indéformable qui peut être attaché autour du membre attenant à l'articulation et qui présente des dispositifs de fermeture (2) par l'intermédiaire desquels le corps de base (10) peut être attaché au membre attenant à l'articulation, un châssis externe (70) indéformable s'étendant sur l'articulation du tarse étant fixé de façon amovible sur le corps de base (10) par l'intermédiaire de moyens de fixation (60, 80) réglables, et le châssis externe (70) s'appuyant sur le membre attenant à l'articulation du tarse, **caractérisé en ce que** des affaiblissements de matériau sont formées dans le corps de base (10) le long de lignes de courbure ou de pliage (17) et le corps de base (10) présente au moins un élément de liaison par formes conjuguées (50) agencé extérieurement, pour la fixation d'au moins une partie (72) du châssis externe (70).

2. Système d'orthèse pour l'articulation du tarse selon la revendication 1, **caractérisé en ce que** l'élément de liaison par formes conjuguées (50) est formé par une poche, et il est cousu, collé, fixé par fermeture autoagrippante ou soudé.

3. Système d'orthèse pour l'articulation du tarse selon la revendication 1 ou 2, **caractérisé en ce que** le châssis externe (70) est monté déplaçable longitudinalement dans l'élément de liaison par formes conjuguées (50).

4. Système d'orthèse pour l'articulation du tarse selon l'une quelconque des revendications précédentes, **caractérisé en ce que** des dispositifs de logement (90) pour des éléments de raidissement sont formés sur ou dans le corps de base (10) par des poches.

5. Système d'orthèse pour l'articulation du tarse selon la revendication 4, **caractérisé en ce que** les éléments de raidissement sont agencés pour limiter au moins une direction de mouvement de l'articulation du tarse.

6. Système d'orthèse pour l'articulation du tarse selon l'une quelconque des revendications précédentes, **caractérisé en ce qu'**une première partie (72) du châssis externe (70) s'étend le long d'une jambe inférieure et une deuxième partie (71) du châssis externe (70) s'étend dans la région plantaire d'un pied, **en ce que** la première et la deuxième partie (72, 71) sont agencées l'une par rapport à l'autre selon un angle approximativement droit, et **en ce que** la deuxième partie (71) laisse libre la première métaphyse distale.

7. Système d'orthèse pour l'articulation du tarse selon la revendication 6, **caractérisé en ce que** la deuxième partie (71) peut se courber par rapport à la première partie (72), dans une position de pronation et/ou une extension dorsale.

8. Système d'orthèse pour l'articulation du tarse selon l'une quelconque des revendications précédentes, **caractérisé en ce que** des encoches (13) sont formées dans le corps de base (10) dans la région du tendon d'Achille, par l'intermédiaire desquelles sont formées des ailettes d'adaptation (14).

9. Système d'orthèse pour l'articulation du tarse selon l'une quelconque des revendications précédentes, **caractérisé en ce que** le corps de base (10) présente un rembourrage sous la forme d'une couche molletonnée, **en ce que** le corps de base (10) est entièrement recouvert du côté extérieur par une couche molletonnée (20) et le rembourrage et/ou la couche molletonnée (20) extérieure sont collées ou soudées sur le corps de base (10).

10. Système d'orthèse pour l'articulation du tarse selon la revendication 9, **caractérisé en ce que** le corps de base (10) présente des évidements (16) à l'intérieur de son contour, à travers lesquels le rembourrage et la couche molletonnée (20) sont collées ou soudées ensemble, en particulier sont soudées au laser ou soudées à haute fréquence.

11. Système d'orthèse pour l'articulation du tarse selon l'une quelconque des revendications précédentes, **caractérisé en ce que** le corps de base (10) est formé symétrique en miroir.

12. Système d'orthèse pour l'articulation du tarse selon l'une quelconque des revendications précédentes, **caractérisé en ce que** les moyens de fixation (60, 80) sont formés par des sangles de traction semi-élastiques ou non élastiques, fixables au corps de base (10).
